# EUROPEAN PATENT APPLICATION

(11) **EP 4 778 480 A2**
(43) Date of publication of application: **22.07.2026**
(21) Application number: 25217874.4
(22) Date of filing: 24.11.2025
(51) Int. Cl.: A61B 18/14

(54) **PLANAR END EFFECTOR FOR ATRIAL FIBRILLATION ABLATION BY PULSE FIELD ABLATION**

(30) Priority: 31.12.2024 US 202419006746
(71) Applicant: Biosense Webster (Israel) Ltd., 2066717 Yokneam (IL)
(72) Inventor: VAN NIEKERK, Pieter Emmelius, Irvine, 92618 (US); BASU, Shubhayu, Irvine, 92618 (US); HIGHSMITH, Debby, Irvine, 92618 (US); SUAREZ, Paul, Irvine, 92618 (US); BAR-TAL, Meir, 2066717 Yokneam (IL); BERGER, Abraham, 2066717 Yokneam (IL); BERGER, Omer, 2066717 Yokneam (IL)
(74) Representative: Carpmaels & Ransford LLP

(57) **Abstract**

A catheter is presented having a planar end effector with elongated ablation electrodes configured to provide electrical signals to achieve IRE in target tissue. The planar end effector can be aligned along a longitudinal axis of a shaft of the catheter and configured to flex away from the longitudinal axis when making contact with tissue. Bipolar electrical signals can be applied between the elongated ablation electrodes in various pair combinations to achieve PFA. The end effector may also include diagnostic electrodes configured to receive electrical signals from tissue to map tissue, pairs of tissue contact electrodes to determine which portions of the end effector are in contact with tissue, or reference electrodes configured to measure blood voltage.

## Description

### FIELD

The present invention relates generally to medical devices, and in particular to catheters configured to ablate tissue by pulse field ablation.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Arrhythmia is treated surgically or by catheter ablation to cease or modify the propagation of unwanted electrical signals from one portion of the heart to another.

Many contemporaneous ablation approaches utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that can reduce some thermal risks associated with RF ablation but may present tissue damage due to the very low temperature nature of such devices. Maneuvering cryoablation devices and selectively applying cryoablation is generally more challenging compared to RF ablation; therefore, cryoablation is not viable in certain anatomical geometries which may be reached by RF ablation devices.

Pulse field ablation (PFA) is a comparatively new non-thermal method of ablating cardiac tissue by causing irreversible electroporation (IRE) in cells of target tissue. To achieve IRE, short pulses of high voltage electrical signals are delivered to tissues and the electrical signals generate an unrecoverable permeabilization of cell membranes. Examples of systems and devices configured for IRE ablation are disclosed in U.S. Patent Pub. No. 2021/0169550A1, 2021/0169567A1, 2021/0169568A1, 2021/0196372A1, 2021/0177503A1, and 2021/0186604A1, and U.S. Pat. No. 11,540,877 each of which are incorporated herein by reference in their entireties and attached in the Appendix hereto.

Regions of cardiac tissue can be mapped by a catheter to identify the abnormal electrical signals. Some catheter ablation procedures especially those with persistent atrial fibrillation may be performed using electrophysiology (EP) mapping to target areas of aberrant electrical signals. Such EP mapping may include the use of diagnostic electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein in its entirety by reference. Examples of EP mapping catheters are described in U.S. Patent No. 9,907,480, U.S. Patent Pub. No. 2018/0036078, and U.S. Patent Pub. No. 2018/0056038, each of which are incorporated herein by reference in their entireties.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif.

### SUMMARY

A catheter is presented having a planar end effector with elongated ablation electrodes configured to provide electrical signals to achieve IRE in target tissue. The planar end effector can be aligned along a longitudinal axis of a shaft of the catheter and configured to flex away from the longitudinal axis when contacting tissue. Bipolar electrical signals can be applied between the elongated ablation electrodes in various pair combinations to achieve PFA. The end effector may also include diagnostic electrodes configured to receive electrical signals from tissue to map tissue, pairs of tissue contact electrodes to determine which portions of the end effector are in contact with tissue, reference electrodes configured to measure blood voltage, or combination thereof. The end effector may provide PFA to create a lesion with a depth of approximately 7.5 mm in a potato model.

An example end effector of a medical probe includes a planar body and a plurality of ablation electrodes. The planar body has a first side and a second side opposite the first side and extends along a longitudinal axis. The plurality of ablation electrodes includes a first ablation electrode disposed on the first side of the planar body and a second ablation electrode. The end effector is configured to provide bipolar pulse filed ablation electrical signals between the first ablation electrode and the second ablation electrode. The first ablation electrode and the second ablation electrode each have a respective length of at least half of a total length of the end effector as measured from a distal end of the end effector to a distal end of a shaft of the medical probe. The respective lengths of the first ablation electrode and the second ablation electrode define a distal portion of the end effector.

An example method of providing PFA electrical signals to a planar end effector includes: providing a first bipolar pulse field ablation electrical signal between a first ablation electrode and a second ablation electrode, the first ablation electrode being disposed on a first side of a planar body of the planar end effector, and the second ablation electrode being disposed on a second side of the planar body and non-overlapping with the first ablation electrode; and creating, with the planar end effector, a lesion with a depth of approximately 7.5 mm in a potato model.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements. The figures depict one or more implementations of the inventive devices, by way of example only, not by way of limitation.
Figure 1 is an illustration of an example catheter-based electrophysiology mapping and ablation system.
Figure 2 is an illustration of a distal portion of a catheter including an example end effector.
Figure 3 is an illustration of a magnified view of the example end effector illustrated in Figure 2.
Figure 4 is an illustration of a first example electrode configuration for the example end effector including elongated serpentine ablation electrodes and diagnostic electrodes positioned alongside the ablation electrodes.
Figure 5 is an illustration of a second example electrode configuration for the example end effector including elongated ablation electrodes which are respectively segmented into small parallel stripes and diagnostic electrodes positioned alongside the ablation electrodes.
Figure 6 is an illustration of a third example electrode configuration for the example end effector including rectangular elongated ablation electrodes without diagnostic electrodes.
Figure 7 is an illustration of a fourth example electrode configuration for the example end effector including rectangular elongated ablation electrodes interrupted by diagnostic electrodes.
Figure 8 is an illustration of an example electrode configuration on one side the example end effector including elongated ablation electrodes in two ablation electrode regions, diagnostic electrodes positioned in the two ablation electrode regions, tissue contact electrodes positioned between the two ablation electrode regions, and a reference electrode positioned in a proximal region of the illustrated side of the end effector.
Figures 9A, 9B, 9C, and 9D are illustrations of alternative configurations of insulating material in the end effector cross-section in which Figure 9A is configured with openings in the end effector as indicated in Figure 8; Figure 9B includes a polymeric body region extending a width of the end effector; Figure 9C includes a high dielectric layer between each side and extending a width of the end effector; and Figure 9D includes high dielectric tiles between each side and configured to overlap to collapse the end effector into a delivery sheath.
Figure 10 is an illustration of the end effector in which the ablation electrodes are configured to provide 1,200 V pulses between ablation electrode regions.
Figure 11 is an illustration of the end effector applying the treatment illustrated in Figure 10 to a potato model.
Figures 12A and 12B illustrate a voltage applied across electrode pairs in a cross-body configuration in which, for each pair, one ablation electrodes is in contact with tissue and the other electrode is on an opposite side of the end effector body and across a central axis of the end effector body from the first electrode region. Figures 12A and 12B show two different electrode pairings.
Figure 12C illustrates a voltage applied between electrode segments which function as separately activated electrodes.
Figure 13 illustrates a voltage applied across ablation electrode pairs in which both ablation electrodes in the pair are in contact with tissue and positioned across the central axis from each other.
Figure 14 illustrates a voltage applied across ablation electrodes in contact with tissue to ablation electrodes on an opposite side of the end effector and not in contact with tissue.
Figures 15A and 15B illustrate a voltage applied across electrode pairs in which electrodes of the pair are positioned across the central axis from each other.
Figure 16 is an illustration of an example electrode configuration on one side the example end effector including elongated ablation electrodes in four ablation electrode regions, diagnostic electrodes positioned in at least two of the ablation electrode regions, tissue contact electrodes positioned along the longitudinal axis between ablation electrode regions, and a reference electrode positioned in a proximal region of the illustrated side of the end effector.
Figure 17 is an illustration of an example electrode configuration for the example end effector including elongated serpentine ablation electrodes.
Figure 18 is an illustration of an example electrode configuration for the example end effector including rectangular elongated ablation electrodes.
Figure 19 is an illustration of an example electrode configuration for the example end effector including large area ablation electrodes.
Figure 20 is an illustration of an example electrode configuration for the example end effector in which ablation electrodes wrap around a spine or insulative material of the catheter body.
Figure 21 is an illustration of an example electrode configuration for the example end effector in which ablation electrodes partially wrap around a spine or insulative material of the catheter body.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention. To the extent that any materials incorporated by reference herein contain similar terms but differ in definition or description, it will be appreciated that the definitions and descriptions provided herein are to be used in understanding the technology disclosed herein

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

In addition, as used herein, the terms "patient," "host," "user," and "subject" refer to any human or animal subject and are not intended to limit the systems or methods to human use, although use of the subject invention in a human patient represents a preferred embodiment.

The term "proximal" indicates a location closer to the operator whereas "distal" indicates a location further away to the operator or physician.

As discussed herein, "operator" and "user" can include a doctor, surgeon, technician, scientist, or any other individual or delivery instrumentation associated with delivery of a multi-electrode catheter for treatments disclosed herein.

As discussed herein, the terms "bipolar" and "unipolar" when used to refer to IRE ablation schemes describe ablation schemes which differ with respect to electrical current path and electric field distribution. "Bipolar" refers to an IRE ablation scheme utilizing a current path between two electrodes that are both positioned near an ablation site or inside the organ to be treated; current density and electric flux density is typically, but not necessarily, approximately equal at each of the two electrodes. "Unipolar" refers to an IRE ablation scheme utilizing a current path between two electrodes where one electrode having a high current density and high electric flux density is positioned at a ablation site, and a second electrode or electrodes having comparatively lower current density and lower electric flux density is positioned remotely from the ablation site such as on a patch on the patient's skin usually outside the body.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Each phase of the biphasic and monophasic pulse preferably has a square shape having an essentially constant voltage amplitude during a majority of the phase duration. Phases of the biphasic pulse may be separated in time by an interphase delay.

Alternative apparatus and system features and alternative method steps are presented in example embodiments herein. Each given example embodiment presented herein can be modified to include a feature or method step presented with a different example embodiment herein where such feature or step is compatible with the given example as understood by a person skilled in the pertinent art as well as where explicitly stated herein. Such modifications and variations are intended to be included within the scope of the claims.

A catheter is presented having a planar end effector with elongated ablation electrodes configured to provide electrical signals to achieve IRE in target tissue. The planar end effector can be aligned along a longitudinal axis of a shaft of the catheter and configured to flex away from the longitudinal axis when contacting tissue. In some embodiments, the catheter is compatible with an 8.5 French sheath. In some embodiments, the end effector includes a highly insulative layer separating electrodes on either side of the end effector. In order to facilitate collapse of highly rigid insulators, in some embodiments, the highly insulative layer includes tiles of insulating material that overlap when the end effector is retracted into the sheath.

In some embodiments, the end effector includes four elongated electrode segments on each side of the end effector (total of eight elongated electrode segments). The elongated electrode segments extend from approximate the distal end of the end effector to about half, or more than half of the length of the end effector as measured from its distal end to a proximal end of the end effector. The end effector can include two electrode regions per side of the end effector (four electrode regions total) such that each electrode region includes two of the elongated electrode segments connected within the end effector to function as a singular electrode. Alternatively, each of the electrode segments can be bisected approximately in half resulting in twice as many elongated electrode segments each above half as long as the previously described elongated electrode segments. This embodiment can include four electrode regions per side of the end effector, eight electrode regions total.

An electrical signal including pulses to achieve IRE can be applied between pairs of electrodes to achieve PFA. The ablation electrodes can be paired in a variety of ways and pulses can be applied between different pairs in the same PFA treatment. In some embodiments, the end effector may provide PFA to create a lesion with a depth of approximately 7.5 mm in a potato model. Bipolar electrical signals can be applied between the elongated ablation electrodes in various pair combinations to achieve PFA.

Each ablation electrode can be designed with shape based on trade-off between total edge area, total contact area, and open space for non-ablation electrodes. Applicants recognize that a larger ratio of surface area to perimeter may effectively deliver the ablation energy while reducing the chance of electrical arcing. Applicants recognize that it is desirable to avoid arcing. Conversely, serpentine electrodes increase mechanical flexibility of the paddle. Applicants recognize that flexibility of the electrode may result in improved tissue contact and catheter longevity, but the geometry of the serpentine electrode has a lower area to perimeter ratio compared to a solid electrode.

A challenge with existing ablation catheters, and PFA catheters in particular, is integrating diagnostic electrodes into the end effector assembly so that tissue can be mapped and ablated by the same end effector. In some embodiments, the end effector presented herein may include diagnostic electrodes configured to receive electrical signals from tissue to map tissue in addition to the ablation electrodes. Configured as such, the end effector is configured for both mapping and ablation.

In some embodiments, the end effector includes pairs of tissue contact electrodes to determine which portions of the end effector are in contact with tissue.

In some embodiments, the end effector includes reference electrodes configured to measure blood voltage. The ablation electrodes, diagnostic electrodes, or tissue contact electrodes may be used as a reference electrode when not in contact with tissue. The end effector may additionally or alternatively include one or more dedicated reference electrodes positioned on a portion of the end effector or shaft near the treatment or diagnostic electrodes in a location not likely to be in contact with tissue during a procedure, such as on a proximal portion of the end effector.

Aspects of the catheter, end effector, and related methods and systems are described in relation to the figures as follows.

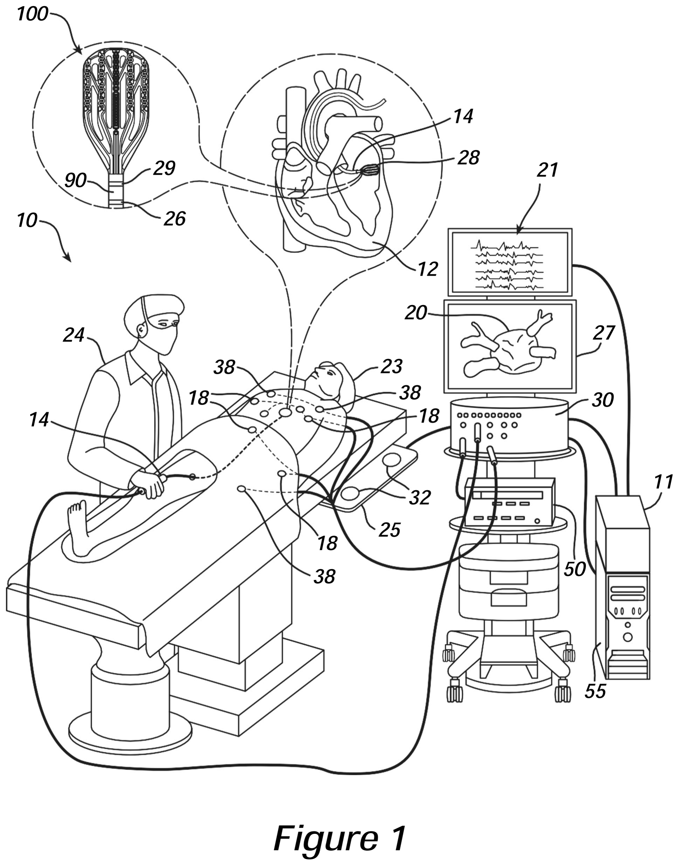

Figure 1 is an illustration showing an example catheter-based electrophysiology mapping and ablation system 10. The system 10 may include multiple catheters, which are percutaneously inserted by a physician 24 through the patient's vascular system into a chamber or vascular structure of a heart 12. Typically, a delivery sheath catheter (e.g. 8.5 French) is inserted into the left or right atrium near a desired location in the heart 12. Thereafter, catheter(s) can be inserted into the delivery sheath catheter so as to arrive at the desired location. Such catheter(s) may include catheters dedicated for sensing Intracardiac Electrogram (IEGM) signals, catheters dedicated for ablating, or catheters dedicated for both sensing and ablating.

An example catheter 14 including an end effector 100 that is configured for providing PFA is illustrated herein. The end effector 100 includes ablation electrodes configured to provide PFA and optionally diagnosis electrodes, tissue contact electrodes, and at least one reference electrode. In some embodiments, the end effector 100 includes diagnostic electrodes configured for mapping aberrant electrical signals in the heart to detect atrial fibrillation and is thus the end effector 100 may be configured for both sensing and mapping. In such examples, the diagnostic electrodes are on the same side of the end effector and in close proximity to the ablation electrodes. The physician 24 brings the end effector 100 into contact with the heart wall for sensing a target site in the heart 12 and providing ablation treatment to the target site without the need to reposition the end effector between a mapping step and an ablation step. Electrode arrangements are shown in greater detail in subsequent figures. Electrodes may be used for more than one purpose as understood by a person skilled in the art informed by the examples presented herein.

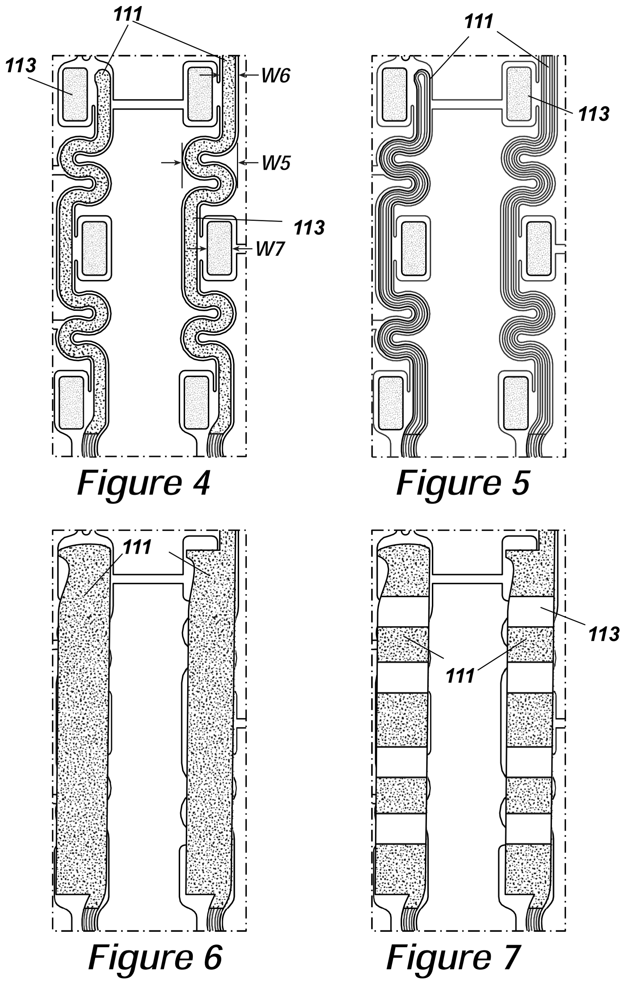

A magnetic based position sensor 29 may be operated together with a location pad 25 including a plurality of magnetic coils 32 configured to generate magnetic fields in a predefined working volume. Real time position of the end effector 100 of the catheter 14 may be tracked based on magnetic fields generated with a location pad 25 and sensed by a magnetic based position sensor 29 disposed in the catheter shaft 90. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated in their entireties by reference herein. The end effector 100 may further include one or more inductive coils configured to provide electrical signals to the magnetic based position sensing system to determine location or orientation of the end effector. For instance, the end effector 100 may include inductive loops or coils similar to as illustrated in Figures 5A and 5B of U.S. Patent Publication No. 2024/0215894 incorporated by reference in its entirety herein and attached in the Appendix hereto. In some embodiments, one or more inductive coils in the end effector may be used together with position sensor 29 to determine location or orientation of the end effector.

The system 10 includes one or more electrode patches 38 positioned for skin contact on the patient 23 to establish location reference for location pad 25 as well as impedance-based tracking of electrode(s) 26. For impedance-based tracking, electrical current is directed toward electrode(s) 26 and sensed at electrode skin patches 38 so that the location of each electrode can be triangulated via the electrode patches 38. The illustration shows an impedance-based tracking electrode 26 affixed to the catheter shaft 90. Additionally, or alternatively, the end effector 100 may include one or more impedance-based tracking of electrode(s). Details of the impedance-based location tracking technology are described in US Patent Nos. 7,536,218; 7,756,576; 7,848,787; 7,869,865; and 8,456,182 incorporated by reference herein in their entireties.

A recorder 11 displays electrograms 21 captured with body surface ECG electrodes 18. In embodiments in which the end effector 100 includes diagnostic electrodes, the recorder 11 may further display intracardiac electrograms (IEGM) derived from electrical signals from diagnostic electrodes on the end effector 100. The recorder 11 may include pacing capability for pacing the heart rhythm or may be electrically connected to a standalone pacer.

The system 10 includes an ablation energy generator 50 that is adapted to conduct ablative energy to ablation electrodes on the end effector 100. Energy produced by the ablation energy generator 50 includes pulses to induce IRE to ablate via PFA and may also be configured to provide radiofrequency (RF) energy to warm tissue to facilitate PFA with no significant thermal ablation or heat tissue to thermally ablate in addition to PFA. The ablation energy generator 50 is preferably configured to provide biphasic bipolar pulses to induce IRE while keeping tissue temperature below thermal ablation temperatures. Additionally, or alternatively the ablation generator 50 is configured to provide monophasic IRE pulses, unipolar IRE pulses, thermal ablation electrical signals, or combinations thereof. For instance, the ablation energy generator 50 can be configured to provide pulses similar to as described in in U.S. Patent Pub. No. 2021/0169550A1, 2021/0177503A1, 2021/0186604A1, and 2023/0009191A1 and U.S. Patent No. 11,540,877B2, each of which are incorporated herein by reference in their entireties and attached in the Appendix hereto. U.S. Patent No. 11,540,877B2 corresponds to U.S. Patent Pub. No. 2021/0161592A1, which is incorporated here by reference in its entirety.

For instance, as described in U.S. Patent No. 11,540,877B2, the generator 50 can be configured to apply bipolar pulses having an amplitude sufficient to cause IRE in the tissue contacted by the electrodes and also RF energy having power sufficient to thermally ablate the tissue contacted by the electrodes. In some embodiments, the sequence of bipolar pulses includes pulses having an amplitude of at least 200 V, and a duration of each of the bipolar pulses is less than 20 µs. Additionally, or alternatively, the RF signal has a frequency between 350 and 500 kHz and an amplitude between 10 and 200 V. The end effector may further include temperature sensors and the electrical signal generator can be configured to apply the signals responsively to a temperature measured by the temperature sensors. In some embodiments, the IRE signal may have parameters as indicated in Table 1 of U.S. Patent No. 11,540,877B2. Note that the "bipolar pulse" as described in U.S. Patent No. 11,540,877B2 is referred as a "biphasic pulse" herein which relates to the shape of an electrical signal; whereas a "bipolar pulse" as described herein relates to the arrangement of electrodes receiving the pulse as defined herein.

A patient interface unit (PIU) 30 is an interface configured to establish electrical communication between catheters, electrophysiological equipment, power supply, and a workstation 55 for controlling operation of system 10. Electrophysiological equipment of the system 10 may include for example, multiple catheters, the location pad 25, body surface ECG electrodes 18, electrode patches 38, the ablation energy generator 50, and the recorder 11. Optionally and preferably, the PIU 30 includes processing capability for implementing real-time computations of location of the catheters and for performing ECG calculations. The PIU 30 can control the generator 50 to provide electrical energy to the ablation electrodes of the end effector 100 according to the ablation protocols described above and in the above incorporated references. The PIU 30, workstation 55, and the generator 50 can collectively be considered an ablation system console having one or more output ports configured to provide ablation energy to the ablation electrodes, one or more processors, and non-transitory computer-readable medium in communication with the processor to cause the ablation system console to provide ablation energy as described above and in the examples herein below.

In some embodiments, the system 10 further includes an irrigation system configured to irrigate during IRE. In some embodiments, the PIU 30 is configured to control the irrigation system to provide irrigation to the catheter end effector similar to as described in U.S. Patent Pub. No. 2021/0196372A1 incorporated by reference in its entirety herein and attached in the Appendix hereto. The irrigation fluid may exit the distal end of the catheter 100 through a port at the distal end of the shaft or through pores in the body of the end effector.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of one or more catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

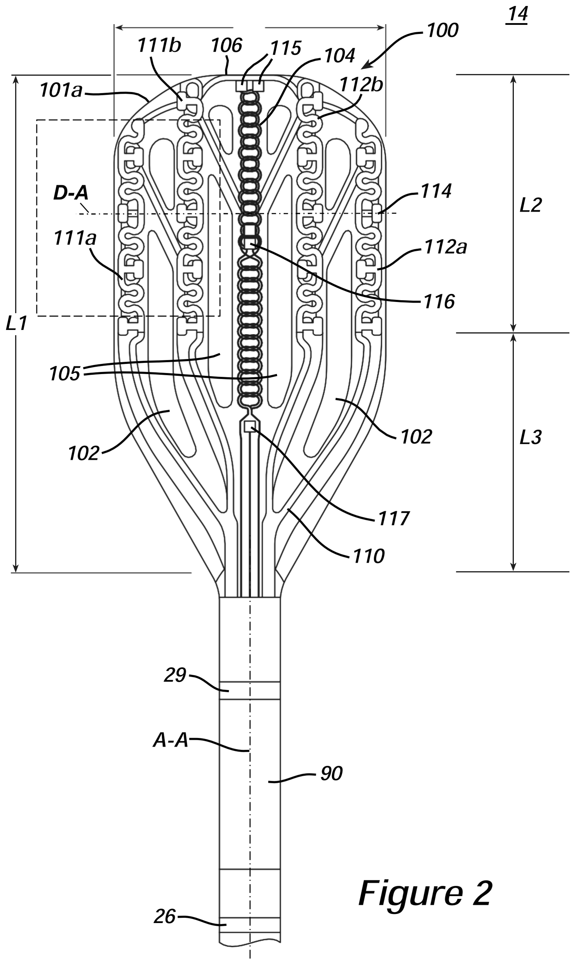

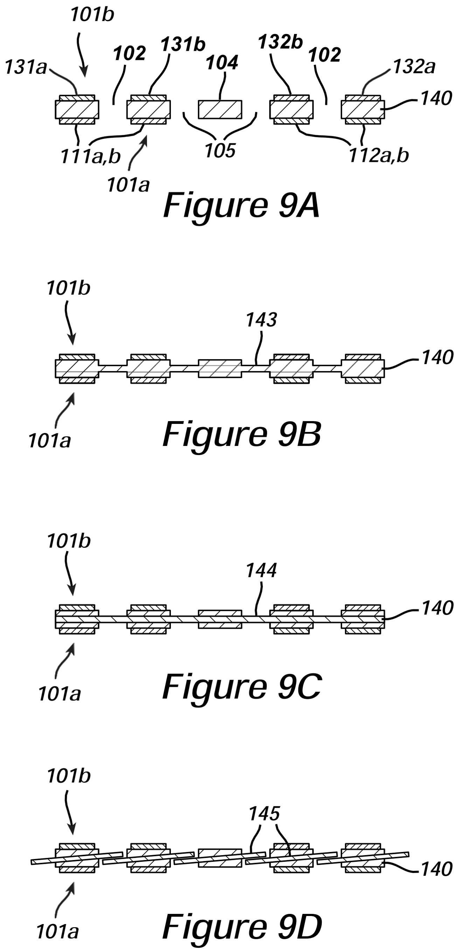

Figure 2 is an illustration of a distal portion of a catheter 14 including an example end effector 100. Figure 2 shows a first side 101a of the end effector 100. The end effector has a planar body 140 with a second side 101b (Figures 9A-9D, 12A-14) opposite the first side. The second side 101b preferably, but not necessarily, has electrodes positioned similar to the first side 101a. When configured with similar electrode configurations on both sides 101a, 101b of the end effector 100, either side can be positioned against tissue during treatment. When one side of the end effector 100 is in contact with tissue, corresponding electrodes on the opposite side are in contact with body fluid (e.g., blood) and can act as respective reference electrodes for those electrodes in contact with tissue.

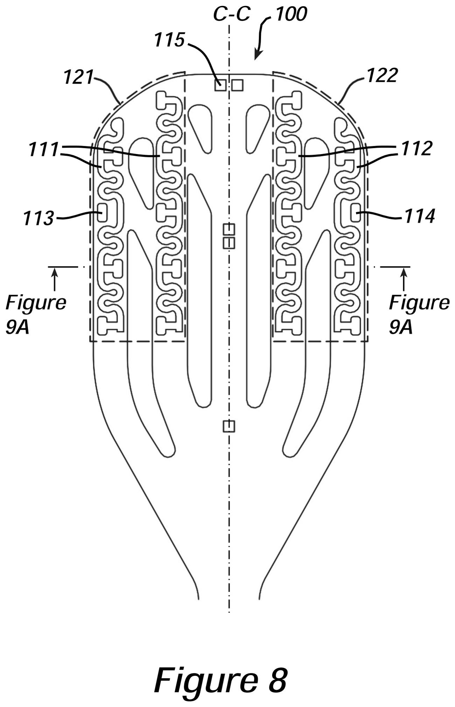

The end effector 100 includes ablation electrodes that each have longitudinally elongated segments 111a, 111b, 112a, 112b that extend along a longitudinal axis A-A defined by the catheter shaft 90. Longitudinally elongated segments 111a, 111b on a left side of the longitudinal axis A-A form a first ablation electrode 111 (Figure 8). Longitudinally elongated segments 112a, 112b on a right side of the longitudinal axis A-A form a second ablation electrode 112 (Figure 8). The ablation electrodes have a length L2 that is at least half of a total length L1 of the end effector 100 as measured from a distal end 106 of the end effector to a distal end of the shaft 90 of the medical probe 14. The ablation electrodes 111, 112 extend to approximately the distal end 106 of the end effector 100. The length L2 of the ablation electrodes define a distal portion of the end effector 100. The end effector 100 has a width W1 measured from leftmost and rightmost edges of the end effector 100. As illustrated, the leftmost and rightmost edges of the end effector 100 define parallel side edges of the end effector 100. The planar body 140 is preferably aligned with the longitudinal axis A-A when in an unconstrained configuration as illustrated in Figure 2.

The ablation electrodes 111, 112 are configured to provide ablation energy to tissue as described in greater detail elsewhere herein. The second side 101b of the planar body 140 of the end effector 100 preferably includes ablation electrodes having longitudinal elongated segments opposite the illustrated longitudinally elongated segments 111a, 111b, 112a, 112b electrodes with respect to a plane defined by the planar body 140. In some embodiments, the ablation electrodes 111, 112 are flush with the planar body 140 to provide a first planar surface to the end effector corresponding to the first side 101a of the planar body and a second planar surface to the end effector corresponding to the second side 101b of the planar body.

In some embodiments, the ablation electrodes on the second side 101b can function as respective reference electrodes for the respective opposite ablation electrode on the first side 101a when the first side 101a is in contact with tissue, and vice versa. Additionally, or alternatively, the ablation electrodes on opposite sides 101a, 101b can be paired to provide bipolar PFA electrical signals between the paired ablation electrodes. Additionally, or alternatively, ablation electrodes on the same side can be paired to provide bipolar PFA electrical signals between the paired ablation electrodes. The bipolar PFA electrical signals may be monophasic or biphasic.

The illustrated end effector 100 includes optional diagnostic electrodes 113, 114 on the first side 101a that are electrically isolated from the ablation electrode segments 111a, 111b, 112a, 112b. The diagnostic electrodes 113, 114 are configured to receive electrical signals from tissue to map cardiac tissue and detect arrhythmia. As illustrated, the diagnostic electrodes 113, 114 are arranged as a pair with a first diagnostic electrode 113 on the left side of the longitudinal axis A-A and a second diagnostic electrode 114 on a right side of the longitudinal axis A-A. The diagnostic electrodes 113, 114 are disposed diametrically along an axis D-A that is orthogonal to the longitudinal axis A-A with respect to each other. The second side 101b of the planar body 140 of the end effector 100 preferably includes a second pair of diagnostic electrodes opposite the first pair of diagnostic electrodes with respect to a plane defined by the planar body 140.

The illustrated end effector 100 includes optional tissue contact quality electrodes 115, 116 positioned in closely spaced pairs such that impedance measured across the respective tissue contact quality electrode pair indicates that electrodes of that pair are both in contact with tissue. As illustrated, the first side 101a includes a distal pair of tissue contact quality electrodes 115 disposed approximate the distal end 106 of the end effector 100 and a central pair of tissue contact quality electrodes 116 positioned approximately in the middle of the length L2 of the distal portion of the end effector 100. The pairs of tissue contact quality electrodes 115, 116 are spaced apart along the longitudinal axis A-A. The planar body 140 of the end effector includes a center portion 104 extending along the longitudinal axis A-A and centrally along the total length L1 that lacks any ablation electrode. As illustrated, the tissue contact quality electrodes 115, 116 are positioned in this central portion 104. The second side 101b of the planar body 140 of the end effector 100 preferably includes corresponding tissue contact quality electrode pairs opposite the pairs of tissue contact quality electrodes 115, 116 on the first side 101a with respect to a plane defined by the planar body 140.

The illustrated end effector 100 includes an optional reference electrode 117 disposed on the first side 101a of the planar body 140. Additionally, or alternatively, the end effector 100 may include a reference electrode similarly disposed on the second side 101b of the planar body 140. The reference electrode(s) 117 may be used for ECG gathering in either, unipolar, bipolar (split or close pair) or may be a reference. The number can be as few as 4 per side but as many as needed (and can fit with trace limitations). The signals from the reference electrode(s) 117 may also be used for contact information to determine what portion of the paddle has contact or proximity to the tissue. The end effector 100 includes a proximal portion defined by a proximal length L3 which lacks any ablation electrode. The reference electrode 117 may be disposed in this proximal portion. As discussed herein, a ablation electrode, diagnostic electrode, or tissue contact electrode may be used as a reference electrode for a corresponding electrode on the opposite side in contact with tissue. Each of these electrodes are positioned in the distal portion of the end effector 100 defined by the ablation electrode length L2 so that they may be positioned in contact with tissue if so desired by the physician 24 (Figure 1). Preferably, the reference electrode 117 is positioned such that the physician 24 is unable, or at least very unlikely, to position the reference electrode 117 in contact with tissue but nevertheless in relatively close proximity to electrodes which are configured to contact tissue. For instance, the end effector 100 may be configured to flex such that while the distal portion of the first side 101a is in contact with tissue, a majority of the proximal portion is not in contact with tissue. By being positioned in the proximal portion, the reference electrode 117 is therefore unable or unlikely to contact tissue during a procedure. Said another way, the end effector is configured to flex such that a majority of the first side 101a of the distal portion is configured to conform to a planar surface while the reference electrode is separated from the planar surface. In such embodiments, the illustrated reference electrode 117 is a dedicated reference electrode rather than a multi-purpose electrode.

In some embodiments, at least the ablation electrodes, diagnostic electrodes, and tissue contact electrodes are flush with the planar body to provide a first planar surface to the end effector 100 corresponding to the first side 101a of the planar body 140 and a second planar surface to the end effector 100 corresponding to the second side 101b of the planar body. The ablation electrodes 111, 112 and any combination of other electrodes 113, 114, 115, 116, 117 may include an exposed conductive layer in a flexible printed circuit board, may include silver epoxy, or conductive ink.

The illustrated planar body 140 includes openings 102, 105 between the first side 101a and the second side 101b. The openings 102, 105 can be sized, shaped, or otherwise configured to allow the end effector to flex through a sheath catheter. The opening configuration may be determined by the size of a sheath catheter through which the end effector is configured to be delivered. The openings 102, 105 can be sized, shaped, or otherwise configured to collapse or expand when the end effector is pressed against a non-planar tissue surface to provide conformal contact to the non-planar tissue surface. Applicants recognize that consistent contact of ablation electrodes to the non-planar tissue may produce improved lesions compared to less conformal electrode contact. In some embodiments the distal end may not be fully closed to allow for more flexibility and larger area. In some embodiments EM loops extend through paddle spines between the openings. An outer opening 102 is disposed between elongated ablation electrode segments 111a, 111b, 112a, 112b of respective ablation electrodes 111, 112. An inner opening 105 is disposed between inner elongated ablation electrode segments 111b, 112b and the central portion 104.

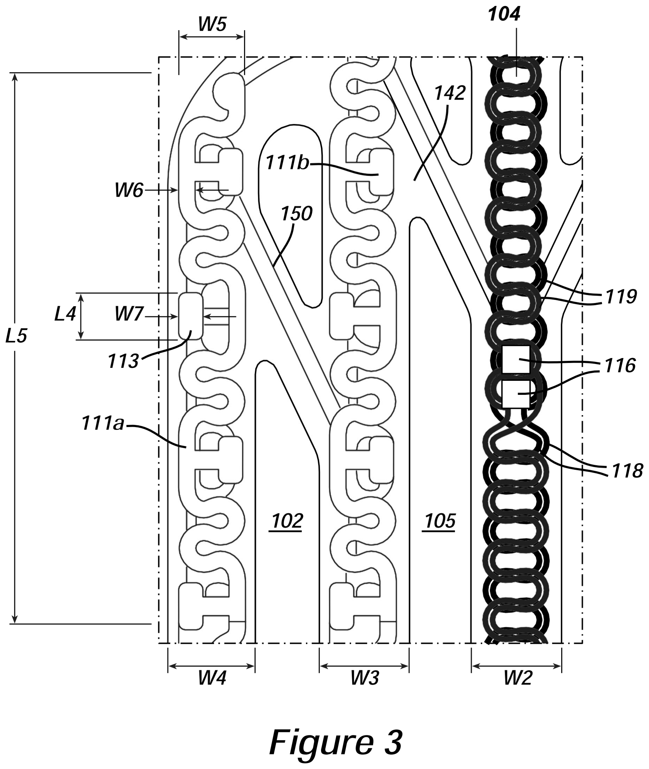

Figure 3 is an illustration of a magnified view of a portion of the example end effector 100 as indicated in Figure 2. The planar body 140 includes a frame 150 surrounded by a flexible, electrically insulating encapsulation 142. The insulating encapsulation may be composed primarily of polymeric material or may include more highly insulative components as described in greater detail in relation to Figures 9A through 9D. The frame 150 is resilient and provides structural support for the end effector 100, allowing the end effector to be constricted to a delivery configuration for delivery through a sheath then self-expand to the unconstrained planar configuration as illustrated, flex to allow the distal portion to conform to a planar or approximately planar surface such as tissue, and collapse upon retraction into a sheath. The frame 150 may be formed from a super elastic material or a shape-memory alloy such as nickel-titanium, also known as Nitinol, cobalt chromium, stainless steel, or other alloys that exhibit pseudo-elastic or superelastic properties.

The illustrated portion of the planar body 140 is divided into three segments: an outer segment including an outer elongated ablation electrode segment 111a, an inner segment including an inner elongated ablation electrode segment 111b, and the central portion 104. The planar body 140 includes a second outer segment and a second inner segment on the right side of the planar body shown in Figure 2 but not Figure 3 so that the planar body 140 has a total of five segments. The outer segment has a width W4; the inner segment has a width W3; and the central portion 104 has a width W2. In the illustrated example, the body segment widths W2, W3, W4 are approximately equal to each other. Each elongated ablation electrode segment 111a, 111b has a width W5 that is more than half, approximately 80% of the width of the outer body segment width W4 and the inner body segment width W3. The ablation electrode segment width W5 is approximately 10% of the total width W1 of the end effector 100.

The elongated ablation electrode segments 111a, 111b are positioned left or right of each other. Each of the elongated ablation electrode segments 111a, 111b extends distally to approximately a distal edge of the planar body 140. Because the distal edge is curved, the inner ablation electrode segment 111b defines the length L2 of the distal portion, while the length L5 of the outer ablation electrode segment 111a is shorter than the length L2 of the inner ablation electrode segment 111b.

The illustrated diagnostic electrode 113 is positioned on the outer segment of the planar body 140 and entirely within the width W5 and length L5 of the outer longitudinally extending ablation electrode segment 111a. The diagnostic electrode 113 has a width W7 that is less than half, preferably approximately one third, the width W5 of the outer longitudinally extending ablation electrode segment 111a. The diagnostic electrode 113 has a length L4 less than half, less than or about one fifth, or less than or about one tenth, the length L5 of the outer longitudinally extending ablation electrode segment 111a.

Each elongated ablation electrode segment 111a, 111b has a serpentine shape. The serpentine shape of the outer longitudinally extending ablation electrode segment 111a bends around three of the four sides of the diagnostic electrode 113. As illustrated, the serpentine shape has a path width W6 that is approximately equal to the width W7 of the diagnostic electrode W7 and approximately one third of the width W5 of the outer longitudinally extending ablation electrode segment 111a.

The end effector 100 further includes electrical traces 118, 119 having an insulated, serpentine-shaped electrical conductor portion. The illustrated electrical traces 118, 119 extend longitudinally, forming a serpentine path within the central portion 104. The illustrated electrical traces 118, 119 make electrical connection to the central pair of tissue contact quality electrodes 116 and the distal pair of tissue contact quality electrodes 115.

Figure 4 is an illustration of a first alternative electrode configuration for the example end effector 100 including one example configuration of a ablation electrode 111 and diagnostic electrodes 113. Similar to Figures 2 and 3, the ablation electrode 111 includes serpentine longitudinally extending segments with a total width W5 and a path width W6. While the configuration in Figures 2 and 3 includes only a single diagnostic electrode 113 per ablation electrode 111, the example in Figure 4 includes six diagnostic electrodes 113 per ablation electrode 111. The end effector 100 may include one, two, three, four, five, or six diagnostic electrodes 113 per ablation electrode 111. Each diagnostic electrode is similarly sized with a width W7 that is approximately one and a half times the path width W6 of the ablation electrode 111 and approximately one half the total width W5 of an elongated segment of the ablation electrode 111. The diagnostic electrodes 113 are surrounded on two or three sides by a respective elongated segment of the ablation electrode 111.

Figure 5 is an illustration of a second alternative electrode configuration for the example end effector including ablation electrodes 111 having elongated segments which are respectively segmented into small parallel stripes and diagnostic electrodes 113 positioned alongside the elongated ablation electrode segments. Each of the elongated segments has multiple electrically conductive stripes running parallel to each other to form an overall shape of a respective elongated segment that is similar to as shown in Figure 4. Other ablation electrode shapes illustrated herein, and alternatives thereto as understood by a person skilled in the pertinent art may also be divided into multiple electrically conductive stripes running parallel to each other similar to as shown in Figure 5. This configuration increases the total edge length of the ablation electrode to provide a different electric field profile to tissue during PFA than a similarly shaped ablation electrode that is solid (e.g., as shown in Figure 4).

Figure 6 is an illustration of a third example electrode configuration for the example end effector including rectangular elongated segments of the ablation electrode 111 without diagnostic electrodes 113. The entire width W5 of the electrode segment is utilized for the ablation electrode 111 to maximize surface area of the ablation electrode 111. Diagnostic electrodes may be disposed elsewhere on the planar body, for instance in the central portion 104.

Figure 7 is an illustration of a fourth example electrode configuration for the example end effector including rectangular elongated segments of the ablation electrode 111 interrupted by diagnostic electrodes 113. Compared to the continuous rectangular electrode segments shown in Figure 6, the segmented elongated ablation electrode segments can have greater flexibility for the planar body 140 to flex away from the longitudinal axis A-A at the expense of total ablation electrode area.

Figure 8 is an illustration of an example electrode configuration on one side the example end effector including elongated ablation electrodes 111, 112 in two ablation electrode regions 121, 122, diagnostic electrodes 113, 114 positioned in the two ablation electrode regions 121, 122, tissue contact electrodes 115, 116 positioned between the two ablation electrode regions 121, 122, and a reference electrode 117 positioned in a proximal region of the illustrated side of the end effector. In some embodiments, the total area of each ablation electrode 111, 112 within a respective electrode region 121, 122 is approximately 7.5 mm². In Figure 8, it can be seen that there are two individual ablation electrodes 111a, 111b on the left side and two individual ablation electrodes 112a, 112b on the right side. Left side electrodes 111a and 111b can be connected to the pulse field generator to deliver a biphasic pulse field between the electrodes 111a and 111b. Right side electrodes 112a and 112b can be connected to the pulse field generator to deliver a biphasic pulse field between the electrodes 112a and 112b. Alternatively, each of the left electrodes 111a and 111b can be connected to the generator with the each of the right electrodes 112a and 112b to deliver a bipolar pulse between the left side electrode and the right side electrode. For example, a bipolar electrode pair can be formed by one or more left electrode 111a and 111b with one or more right electrodes 112a and 112b.

A centerline C-C is drawn in Figure 8 which indicates a line of symmetry for an electrode configuration or planar body. As illustrated, the electrode configuration is symmetric about a centerline C-C. Preferably, at least the ablation electrodes 111, 112 are symmetric to each other with respect to the centerline C-C. The planar body may also be a symmetric shape about the centerline C-C as illustrated. In an alternative embodiment, the planar body may not be symmetric about the centerline C-C. As illustrated, the centerline C-C is coextensive with the longitudinal axis A-A. In an alternative embodiment, the centerline C-C may not be coextensive with the longitudinal axis A-A.

The illustrated side of the planar body has a left half which is left of the centerline C-C and a right half which is right of the centerline C-C. The first ablation electrode 111 defines the first electrode region 121, which is entirely in the left half of the planar body 140. The second ablation electrode 112 defines the second electrode region 122, which is entirely in the right half of the planar body 140. The tissue contact electrodes 115, 116 are arranged in pairs that each are disposed across the centerline C-C on the illustrated side of the planar body 140. A distal pair of tissue contact electrodes 115 are approximate the distal end 106 of the planar body 140. A central pair of tissue contact electrodes 116 are in a proximal direction in relation to the distal pair 115 and across the centerline C-C. The reference electrode 117 is disposed on the illustrated side of the planar body 140 and across the centerline C-C.

Figures 9A through 9D are illustrations of alternative configurations of insulating material in the end effector cross-section. A second side 101b of the planar body 140 is indicated opposite the first side 101a. Ablation electrodes 131, 132 are illustrated on the second side 101b opposite the ablation electrodes 111, 112 on the first side. The opposite ablation electrodes 131, 132 overlap the ablation electrodes 111, 112 on the first side 101a, preferably overlapping a majority of the ablation electrodes 111, 112 on the first side 101a, and may be symmetric to the ablation electrodes 111, 112 on the first side 101a with respect to a plane defined by the planar body 140. The cross-section of the planar body 140 is simplified to omit the frame 150, electrical traces, and other such features for the sake of illustration. The ablation electrodes 111, 112, 131, 132 are illustrated projecting from the planar body 140, but may alternatively be sunken into the planar body 140 to provide a smooth planar surface on each respective side 101a, 101b. The ablation electrodes 111, 112, 131, 132 can have approximately equal surface area to each other. Each segment 111a, 111b, 112a, 112b, 131a, 131b, 132a, 132b of the ablation electrodes are shown as contiguous across their width. Alternatively, some or all of the segments can include parallel stripes similar to as illustrated in Figure 5, resulting in a segmented cross-sectional profile.

The various configurations shown in Figures 9A through 9D provide differing levels of electrical insulation between electrodes on opposite sides of the planar body. The electrical insulation may be tailored to direct electric field lines, and thereby electroporation of cells within target tissue, between bipolar pairs of ablation electrodes on opposite sides of the planar body 140 during PFA application.

Figure 9A is configured with openings in the end effector as indicated in Figure 8. Figure 9B includes a polymeric body region extending a width of the end effector. Essentially, some or all of the openings 102, 105 (and other non-labeled openings) of the example end effector 100 illustrated in Figures 2 and 3 can include a respective thinned polymer fill-in region which lacks a framework or any electrical circuitry. Configured as such, the planar body 140 may provide additional electrical insulation between electrodes on opposite sides of the planar body 140 (compared to openings 102, 105) while maintaining sufficient flexibility for manipulation to position against tissue and transfer through a sheath. Figure 9C includes a planar high dielectric layer 144 between each side and extending a width of the end effector. The dielectric layer 144 is overlapping and parallel to each of the ablation electrodes 111, 112, 113, 114. The planar high dielectric layer 144 can include a ceramic doped polymer to provide additional electrical insulation between electrodes on opposite sides of the planar body compared to polymer alone while still providing sufficient flexibility. As illustrated, the planar high dielectric layer 144 extends across a majority of an entire width W1 of the planar body 140. The planar body 140 includes longitudinally elongated regions (e.g. corresponding to openings 102, 105 in Figures 2 and 3) including the planar high dielectric layer 140, lacking a framework, and lacking electrical circuitry. Figure 9D includes high dielectric tiles 145 between each side 101a, 101b and configured to overlap to collapse the end effector 100 into a delivery sheath. The high dielectric tiles 145 can include ceramic plates that extend longitudinally through each segment of the body and overlap between segments such that the overlap corresponds to openings 102, 105 illustrated in Figures 2 and 3. The high dielectric tiles or longitudinally extending ceramic plates may be angled with respect to a plane defined by the planar body 140 such that the tiles/plates 145 are configured to overlap, longitudinal side on longitudinal side upon retraction of the end effector into a sheath.

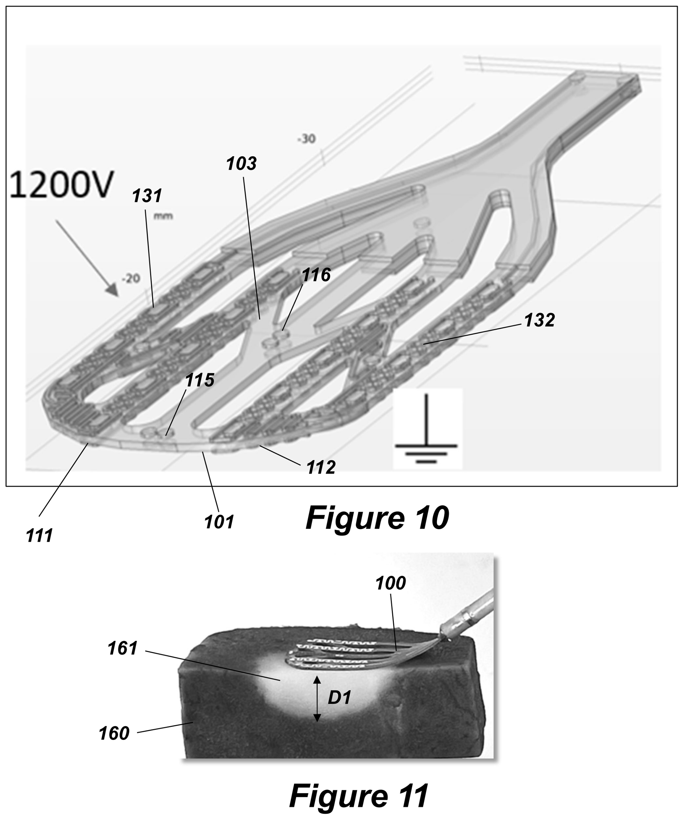

Figure 10 is an illustration of the end effector in which the ablation electrodes are configured to provide 1,200 V pulses between ablation electrode regions. In some embodiments, the end effector 100 is configured to provide PFA electrical pulses having a voltage of about 600 V and about 1,200 V between pairs of ablation electrodes 111, 112, 131, 132. As illustrated in Figure 10, the end effector 100 is configured to provide electrical pulses 1,200 V in amplitude between bipolar pairs which include a ablation electrode on the left side of the planar body and in contact with tissue, i.e., electrode 111 and an electrode on the right side of the planar body and not in contact with tissue, i.e., electrode 132. Similarly, electrodes 112 and 131 are paired. The paired electrodes are on opposite sides of the planar body 140 and non-overlapping.

In some embodiments, cardiac electrical signals may be measured from one or more diagnostic electrode(s) disposed on the side of the planar body in contact with tissue. In some embodiments, tissue contact may be measured from a pair of tissue contact electrodes disposed on the side of the planar body in contact with tissue. In some embodiments, a reference electrical signal may be measured from a reference electrode disposed on the side of the planar body in contact with tissue, wherein the reference electrode itself is not in contact with tissue.

Figure 11 is an illustration of the end effector 100 applying the treatment illustrated in Figure 10 to a potato model 160. The PFA electrical signals applied to the ablation electrodes results in a lesion 161 having a depth D1 of approximately 7.5 millimeters (mm). To create the lesion, a first bipolar PFA electrical signal is applied a first ablation electrode and a second ablation electrode, the first ablation electrode being disposed on a first side of a planar body of the planar end effector, and the second ablation electrode being disposed on a second side of the planar body and non-overlapping with the first ablation electrode (i.e. ablation electrode pair 111, 132). The first bipolar PFA electrical signal includes 60 pulses with a magnitude of approximately 1,200 V and a total duration of approximately 4 seconds. A second bipolar PFA electrical signal is applied between a third ablation electrode and a fourth ablation electrode, the third ablation electrode being disposed on a first side of a planar body of the planar end effector and overlapping with the second ablation electrode, and the fourth ablation electrode being disposed on a second side of the planar body and overlapping with the second ablation electrode, i.e., ablation electrode pair 112, 131. The second bipolar PFA electrical signal includes 60 pulses with a magnitude of approximately 1,200 V and a total duration of approximately 4 seconds. The bipolar pulses may include an interpulse delay of approximately 2 microseconds.

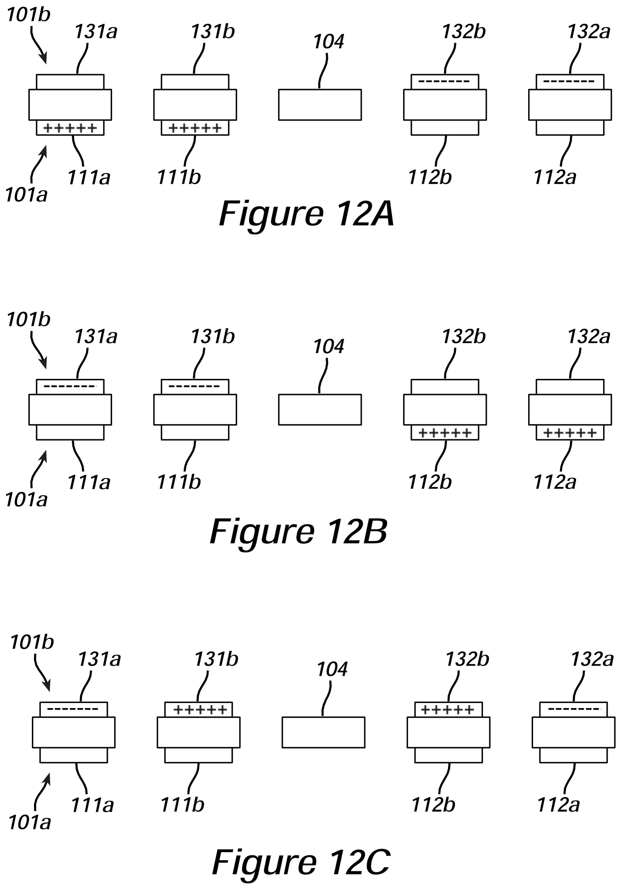

Figures 12A, 12B, 12C, 13, 14, 15A, and 15B illustrate ablation electrode pairing configurations for application of bipolar PFA electrical pulses having a voltage of about 600 V and about 1,200 V in embodiments in which the end effector 100 includes a total four ablation electrodes 111, 112, 131, 132. As described in greater detail in relation to Figure 10, the end effector 100 preferably includes a total of two to eight ablation electrodes. The pattern of ablation electrode pairings can be adapted based on the total number of ablation electrodes in the end effector 100 as discussed in greater detail in relation to Figure 10 and as understood by a person skilled in the pertinent art informed by the disclosure herein.

Figures 12A and 12B illustrate a cross-body pairing to ablation electrodes as described in relation to Figures 10 and 11. Ablation electrodes in a pair are symmetric with respect to the centerline C-C and on opposite sides of the planar body 140. A voltage is applied across electrodes in pairs of electrode regions in which, for each pair, ablation electrodes in a first electrode region is in contact with tissue and the other electrode region is on an opposite side of the end effector and across the center line C-C from the first electrode region. Figures 12A and 12B show two different pairs of electrode regions. Figure 12A illustrates the elongated segments 111a, 111b of ablation electrode 111 having a positive charge while the elongated segments 132a, 132b of ablation electrode 132 have a negative charge. This illustrates a positive voltage pulse configured to induce electroporation in tissue in contact with either side 101a, 101b of the planar body 140. A biphasic pulse may be applied in which the polarity switches or alternates between as shown in Figure 12A to one in which positive charge is on the ablation electrode 132 not in contact with tissue while negative charge is on the ablation electrode 111 in contact with tissue. A train of bipolar pulses (which may include biphasic, monophasic pulses, or combination thereof) can be applied between the bipolar ablation electrode pair 111, 132 as shown in Figure 12A, then subsequently, a train of bipolar pulses can be applied between the bipolar ablation electrode pair 112, 131 shown in Figure 12B.

Figure 12C illustrates another configuration in which ablation electrodes 131a and 131b are considered to be in contact with tissues. In this example, ablation electrodes 131a and 131b are no longer connected to each other but are separately connected to the generator so that a biphasic pulse field can be provided between these two tissue contacting ablation electrodes in a bipolar configuration to allow the flow of electrons between 131a and 131b. Similarly, ablation electrodes 131c and 131d (in tissue contact) are no longer connected to each other but are separately connected to the generator so that a biphasic pulse field can be provided between these two tissue contacting ablation electrodes in a bipolar manner to allow the flow of electrons between them.

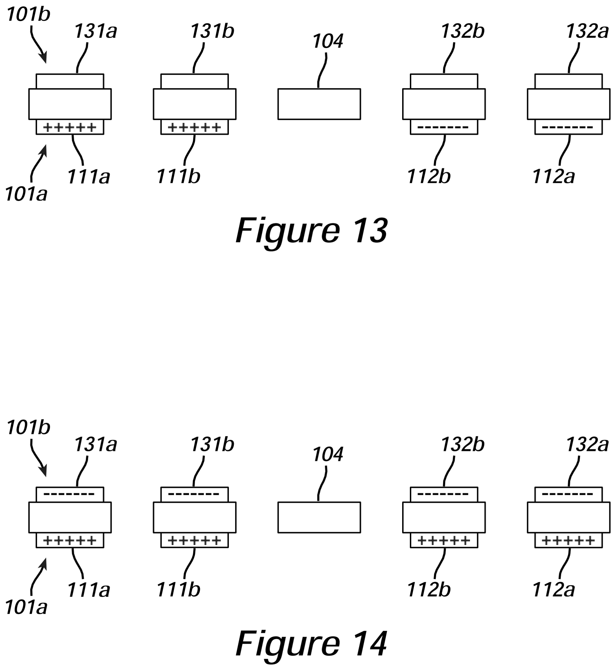

Figure 13 illustrates ablation electrode pairing configuration in which ablation electrodes in the pair are symmetric with respect to the center line C-C and on the same side of the planar body 140. As illustrated, a voltage is applied across electrodes 111, 112 on the side 101a of the planar body 140 in contact with tissue. The electrodes 131, 132 not in contact with tissue may function as reference electrodes. A biphasic pulse may be applied in which the polarity switches or alternates between as shown in Figure 13 to one in which positive charge is on the right ablation electrode 112 while negative charge is on the left ablation electrode 111. A train of bipolar pulses can be applied between the bipolar ablation electrode pair 111, 112.

Figure 14 illustrates a ablation electrode pairing configuration in which ablation electrodes in the pair are overlapping and on opposite sides of the planar body. A voltage is applied across ablation electrodes 111, 112 in contact with tissue to ablation electrodes 131, 132 on an opposite side of the planar body 140 and not in contact with tissue. A biphasic pulse may be applied in which the polarity switches or alternates between as shown in Figure 14 to one in which positive charge is on ablation electrodes 131, 132 not in contact with tissue while negative charge is on ablation electrodes 111, 112 in contact with tissue. A train of bipolar pulses can be applied between the bipolar ablation electrode pairs. As illustrated, both electrodes 111, 112 in contact with tissue are activated simultaneously. Alternatively, a train of bipolar pulses may be applied to the first pair of ablation electrodes while the second pair is deenergized, and subsequently a train of bipolar pulses may be applied to the second pair of ablation electrodes while the first pair is deenergized.

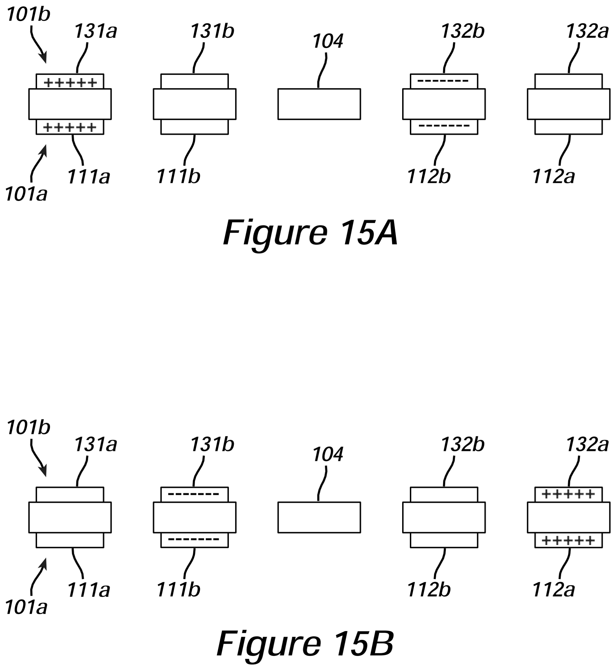

Figures 15A and 15B illustrate a voltage applied across electrode pairs in which electrodes of the pair are positioned across the central axis from each other. The pairs are asymmetric with respect to the centerline such that one electrode (or set of electrodes) in the pair is closer to the centerline than the other. In this configuration, the electrode segments on opposite sides of the end effector body are activated as a unit and may be electrically coupled within the catheter. For example, electrodes 111a and 131a can form a bipolar pair with electrodes 112b and 132b as shown in Figure 15A. Similarly, as shown in Figure 15B, electrodes 111b and 131b can form a bipolar pair with electrodes 112a and 132a. Alternatively, only electrodes on a side of the planar body in contact with tissue may be activated. For instance, ablation electrodes 111a and 112b may form a bipolar pair in Figure 15A while opposite ablation electrodes 131a and 132b are off. Likewise, in Figure 15B, ablation electrodes 111b and 112a may form a bipolar pair in Figure 15A while opposite ablation electrodes 131b and 132a are off. A train of bipolar pulses (which may include biphasic, monophasic pulses, or combination thereof) can be applied between bipolar ablation electrode pairs as shown in Figure 15A and subsequently in Figure 15B, switching back and forth between the bipolar configurations in Figures 15A and 15B.

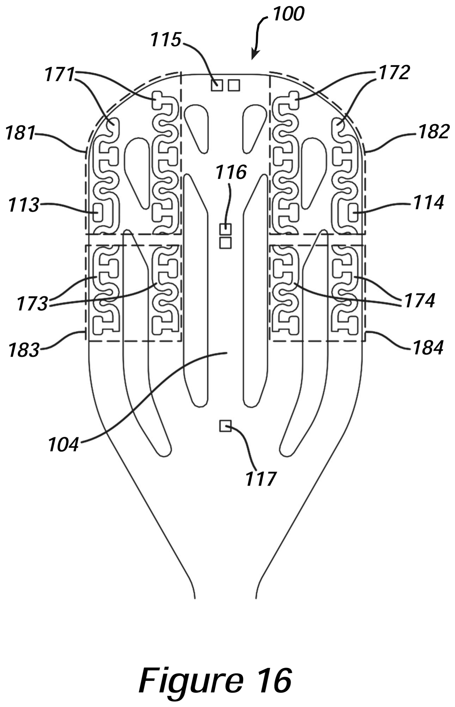

Figure 16 is an illustration of an example electrode configuration on one side the example end effector including elongated ablation electrodes 171, 172, 173, 174 in four ablation electrode regions 181, 182, 183, 184, diagnostic electrodes 113, 114 positioned in at least two of the ablation electrode regions 181, 182, tissue contact electrodes 115, 116 positioned along the centerline C-C between ablation electrode regions, and a reference electrode 117 positioned in a proximal region of the illustrated side of the end effector 100. While embodiments of the end effector 100 described herein above include exactly four ablation electrodes 111, 112, 131, 132; the embodiment illustrated in Figure 15 includes up to eight ablation electrodes including those illustrated 171, 172, 173, 174 and corresponding overlapping ablation electrodes on the opposite side of the planar body not illustrated.

The end effector 100 preferably includes a total of two to eight ablation electrodes. The end effector 100 preferably includes exactly two, three, or four ablation electrodes per side of the planar body 140. Each ablation electrode preferably overlaps a corresponding ablation electrode on the opposite side of the planar body so that the ablation electrodes are symmetric with respect to a plane defined by the planar body 140. Fewer ablation electrodes can be accomplished by electrically connecting combinations of ablation electrodes within the end effector 100 or elsewhere within the catheter 14. Increasing the number of ablation electrodes can be accomplished by splitting apart a ablation electrode into two portions that are electrically insulated from each other in the catheter 14 and configured to be independently activated by the generator 50. Figure 15 shows one example in which the ablation electrode 111 on the left of the centerline C-C as shown in Figure 8 is segmented into two electrodes: an upper left ablation electrode 171 and a lower left ablation electrode 173. Likewise, the ablation electrode 112 on the right of the centerline C-C as shown in Figure 8 is segmented into two electrodes: an upper right ablation electrode 172 and a lower right ablation electrode 174. Preferably, all ablation electrodes 171, 172, 173, 174 have approximately the same surface area as each other.

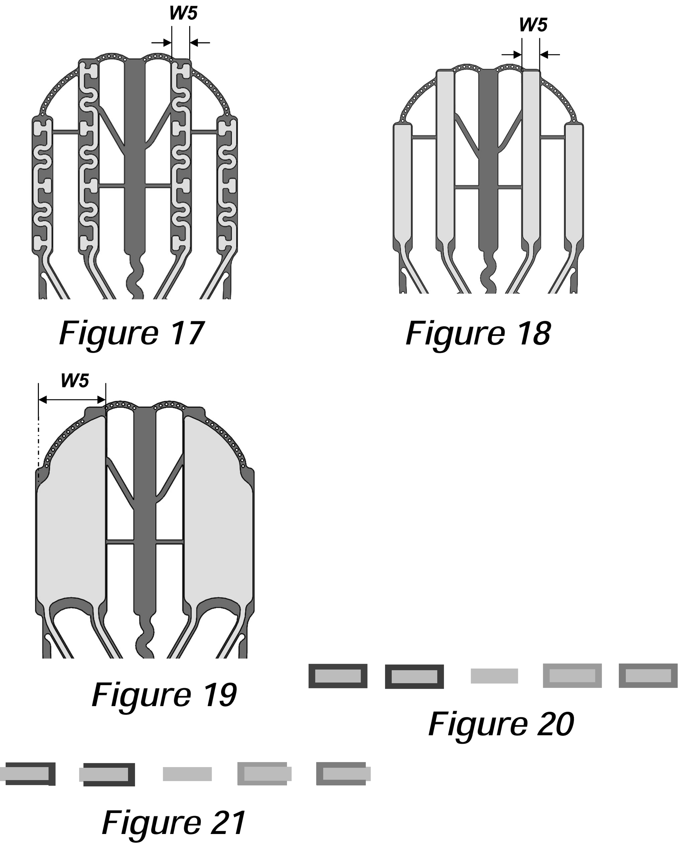

Figure 17 is an illustration of an example electrode configuration for the example end effector including elongated serpentine ablation electrodes. The elongated segments are serpentine similar to as illustrated in Figure 3. In one embodiment, the trace width W6 is approximately 0.28 mm, the electrode segment width W5 is approximately 1 mm, the ablation electrode surface area is approximately 10.1 square mm, the perimeter is approximately 67.7 mm, and the perimeter to area ratio is approximately 7:1.

Figure 18 is an illustration of an example electrode configuration for the example end effector including rectangular elongated ablation electrodes. The elongated segments are solid similar to as illustrated in Figure 6, meaning the trace width is equal to the total width. In one embodiment, the electrode segment width W5 is approximately 1 mm, the ablation electrode surface area is approximately 16.8 square mm, the perimeter is approximately 37.4 mm, and the perimeter to area ratio is approximately 2:1.

Figure 19 is an illustration of an example electrode configuration for the example end effector including large area ablation electrodes. In one embodiment, the electrode segment width W5 is approximately 3.5 mm, the ablation electrode surface area is approximately 28.8 square mm, the perimeter is approximately 24.8 mm, and the perimeter to area ratio is approximately 1:1

Figure 20 is an illustration of an example electrode configuration for the example end effector in which ablation electrodes wrap around a spine or insulative material of the catheter body.

Figure 21 is an illustration of an example electrode configuration for the example end effector in which ablation electrodes partially wrap around a spine or insulative material of the catheter body.

Each electrode in Figures 20 and 21 can be activated separately to form bipolar pairs symmetric or asymmetric with respect to the centerline C-C.

Ablation electrodes can be paired in various pairing combinations to provide bipolar PFA electrical signals between ablation electrodes in a pair. Table 1 includes a summary of selected example ablation electrode pairings based on the illustrated examples herein. Pairs can be activated simultaneously or sequentially in various combinations to achieve PFA of target tissue as understood by a person skilled in the pertinent art informed by the disclosure herein.

**Table 1 Summary of Selected Example Ablation electrode Pairings**

| **Ablation electrode pairing** | **Examples (reference numbers of ablation electrodes in a bipolar pair with "opposite" indicating electrode on the opposite side of planar body than what is illustrated in** **Figure 16****)** |
|---|---|
| Symmetrically across the center line C-C and on opposite sides of the planar body | (111, 132) in Figure 12A; (112, 131) in Figure 12B; (171, opposite 172) in Figure 16; (173, opposite 174) in Figure 16; (172, opposite 171) in Figure 16; and (174, opposite 173) in Figure 16 |
| Symmetrically across the center line C-C and on same side of the planar body | (111, 112) in Figure 13; (131, 132) opposite the planar body as shown in Figure 13 (when in contact with tissue); (171, 172) in Figure 16; and (173, 174) in Figure 16 |
| Overlapping and on opposite sides of the planar body | (111, 131) in Figure 14; (112, 132) in Figure 14; (171, opposite 171) in Figure 16; (172, opposite 172) in Figure 16; (173, opposite 173) in Figure 16; (174, opposite 174) Figure 16 |
| On the same side of the center line C-C and on the same side of the planar body | (171, 173); and (172, 174) in Figure 16 |
| On the same side of the center line C-C, non-overlapping, and on opposite sides of the planar body | (171, opposite 173); (173, opposite 171); (172, opposite 174); and (174, opposite 172) in Figure 16 |
| Asymmetrically across the center line C-C and on opposite sides of the planar body | (171, opposite 174); (173, opposite 172); (172, opposite 173); and (174, opposite 171) in Figure 16 |
| Asymmetrically across the center line C-C and on the same side of the planar body | (171, 174); and (172, 173) in Figure 16; (111a, 112b); (111b, 112a) in Figures 15A and B |

In another alternative embodiment (not illustrated), the segments 111a, 111b of the left ablation electrode 111 illustrated in Figure 8 may be electrically insulated from each other to form separate ablation electrodes. An embodiment in which all elongated ablation electrode segments form independent electrodes, the end effector 100 includes a total of eight elongated ablation electrodes. The example ablation electrode configurations illustrated and described herein are non-limiting and numerous other ablation electrode configurations are possible. In each ablation electrode configuration, ablation electrodes can be paired following the same concepts as outlined in Table 1.

The following clauses list non-limiting embodiments of the disclosure:
Clause 1. An end effector of a medical probe, the end effector comprising: a planar body comprising a first side and a second side opposite the first side and extending along a longitudinal axis; and a plurality of ablation electrodes and comprising a first ablation electrode disposed on the first side of the planar body and a second ablation electrode, the end effector being configured to provide bipolar pulse filed ablation electrical signals between the first ablation electrode and the second ablation electrode, the first ablation electrode and the second ablation electrode each having a respective length of at least half of a total length of the end effector as measured from a distal end of the end effector to a distal end of a shaft of the medical probe such that the respective length of the first ablation electrode and the second ablation electrode define a distal portion of the end effector.
Clause 2. The end effector of clause 1, further comprising: at least one diagnostic electrode disposed on at least one of the first side or second side of the planar body, the at least one diagnostic electrode being electrically isolated from the plurality of ablation electrodes, and configured to receive electrical signals from tissue.
Clause 3. The end effector of clause 2, the at least one diagnostic electrode comprises plural pairs of diagnostic electrodes with each pair being disposed diametrically along an axis orthogonal to the longitudinal axis with respect to each other on respective first and second sides.
Clause 4. The end effector of any one of clauses 1-3, further comprising: a pair of tissue contact electrodes disposed on the first side of the planar body.
Clause 5. The end effector of any one of clauses 1-4, further comprising: a plurality pairs of tissue contact electrodes spaced apart along the longitudinal axis, each pair of the plurality of pairs of tissue contact electrodes being disposed on at least one of the first side or the second side proximate the distal portion of the end effector.
Clause 6. The end effector of any one of clauses 1-4, wherein the plurality of first ablation electrodes comprises four individual first ablation electrodes in which each of the four first ablation electrodes can be connected to the other three ablation electrodes to form one or more combined first ablation electrodes with a greater electrode area than any individual first ablation electrode, and the plurality of second ablation electrodes comprises four individual second ablation electrodes in which each of the four second ablation electrode can be connected to any of the other three second ablation electrodes to form a combined second ablation electrode with a greater electrode area than any individual second ablation electrode.
Clause 7. The end effector of any one of clauses 1-6, the end effector comprising a proximal portion proximal of the distal portion and lacking any ablation electrode.
Clause 8. The end effector of clause 7, in which any two of the individual first ablation electrodes are configured to be electrically connected to a biphasic pulse field generator and any two of the individual second ablation electrodes can be electrically connected to a biphasic pulse field generator, the end effector further comprising: a reference electrode disposed on the first side of the planar body and in the proximal portion.
Clause 9. The end effector of clause 8, wherein the end effector is configured to flex such that a majority of the first side of the distal portion is configured to conform to a planar surface while the reference electrode is separated from the planar surface.
Clause 10. The end effector of any one of clauses 1-9, the first ablation electrode and the second ablation electrode each extending to approximately a distal end of the end effector.
Clause 11. The end effector of any one of clauses 1-10, the planar body comprising a center portion extending along the longitudinal axis and centrally along a total length of the end effector as measured from a distal end of the end effector to a distal end of a shaft of the medical probe, the center portion lacking any ablation electrode.
Clause 12. The end effector of any one of clauses 1-11, first ablation electrode comprising a plurality of longitudinally elongated segments positioned left or right of each other.
Clause 13. The end effector of clause 12, each segment of the plurality of longitudinally extending segments defining a width, wherein a diagnostic electrode is positioned entirely within the width of a respective longitudinally extending segment of the first ablation electrode.
Clause 14. The end effector of clause 13, wherein the diagnostic electrode comprises a width less than half the width of the respective longitudinally extending segment.
Clause 15. The end effector of clause 14, wherein the width of the diagnostic electrode is approximately one third the width of the respective longitudinally extending segment.
Clause 16. The end effector of any one of clauses 12-15, wherein a diagnostic electrode is positioned entirely within a length of a respective longitudinally extending segment of the first ablation electrode.
Clause 17. The end effector of clause 16, wherein the diagnostic electrode comprises a length less than one half the length of the respective longitudinally extending segment.
Clause 18. The end effector of clause 17, wherein the diagnostic electrode comprises a length less than one fifth the length of the respective longitudinally extending segment.
Clause 19. The end effector of clause 18, wherein the diagnostic electrode comprises a length less than one tenth the length of the respective longitudinally extending segment.
20. The end effector of any one of clauses 1-19, wherein each ablation electrode of the plurality of ablation electrodes comprises a serpentine shape.
Clause 21. The end effector of clause 20, wherein the serpentine shape of the first ablation electrode bends around at least three of four sides of the diagnostic electrode.
Clause 22. The end effector of any one of clauses 1-21, wherein each of the plurality of elongated segments comprises a plurality of electrically conductive stripes running parallel to each other to form an overall shape of a respective elongated segment of the plurality of elongated segments.
Clause 23. The end effector of any one of clauses 1-22, further comprising: an electrical trace comprising an insulated, serpentine-shaped electrical conductor portion.
Clause 24. The end effector of any one of clauses 1-23, a total surface area of the first ablation electrode being approximately 7.5 mm².
25. The end effector of any one of clauses 1-24, the plurality of ablation electrodes each being flush with the planar body to provide a first planar surface to the end effector corresponding to the first side of the planar body and a second planar surface to the end effector corresponding to the second side of the planar body.
Clause 26. The end effector of any one of clauses 1-25, further comprising: an inductive loop navigation sensor disposed in the planar body.
Clause 27. The end effector of any one of clauses 1-26, the plurality of ablation electrodes comprising silver epoxy.
Clause 28. The end effector of any one of clauses 1-27, the plurality of ablation electrodes comprising conductive ink.
Clause 29. The end effector of any one of clauses 1-28, the planar body being a symmetric shape about a centerline bisecting the planar body along the longitudinal axis.
Clause The end effector of clause 29, the planar body having a left half and a right half as viewed on the first side, the left half being left of the centerline, the right half being right of the centerline, and the first ablation electrode defining a first electrode region disposed entirely in the left half.
Clause 31. The end effector of clause 30, further comprising: a pair of tissue contact electrodes disposed on the first side of the planar body across the centerline.
Clause 32. The end effector of clause 30 or 31, the pair of tissue contact electrodes being approximate a distal end of the end effector.
Clause 33. The end effector of clause 32, further comprising: a second pair of tissue contact electrodes disposed on the first side in a proximal direction in relation to said pair of tissue contact electrodes and across the centerline.
Clause 34. The end effector of any one of clauses 30-33, further comprising: a reference electrode disposed on the first side of the planar body across the centerline.
Clause 35. The end effector of any one of clauses 30-34, the plurality of ablation electrodes further comprising a third ablation electrode and a fourth ablation electrode, the end effector being configured to provide bipolar pulse filed ablation electrical signals between the third ablation electrode and the fourth ablation electrode.
Clause 36. The end effector of clause 35, the first, second, third, and fourth ablation electrodes having approximately equal surface area to each other.
Clause 37. The end effector of clause 35 or 36, each of the first, second, third, and fourth ablation electrodes defining a respective electrode region, each of the respective electrode regions begin disposed entirely in the right half of the planar body or entirely in the left half of the planar body.
Clause 38. The end effector of any one of clauses 1-37, the planar body comprising openings therethrough between the first side and the second side 101b).
Clause 39. The end effector of any one of clauses 1-38, the planar body comprising a polymer encapsulation.
Clause 40. The end effector of any one of clauses 1-39, the planar body comprising a plurality of elongated thinned polymer fill-in regions lacking a framework and lacking electrical circuitry.
Clause 41. The end effector of any one of clauses 1-40, the planar body comprising a planar high dielectric layer overlapping and parallel to each of the plurality of ablation electrodes.
Clause 42. The end effector of clause 41, the planar high dielectric layer comprising ceramic doped polymer.
Clause 43. The end effector of clause 41 or 42, wherein the planar high dielectric layer extends across a majority of an entire width of the planar body.
Clause 44. The end effector of any one of clauses 41-43, the planar body comprising longitudinally elongated regions comprising the planar high dielectric layer, lacking a framework, and lacking electrical circuitry.
Clause 45. The end effector of any one of clauses 1-44, the planar body comprising at least one ceramic plate.
Clause 46. The end effector of clause 45, the at least one ceramic plate comprising a plurality of longitudinally extending ceramic plates.
Clause 47. The end effector of clause 46, the plurality of longitudinally extending ceramic plates being angled such that the plurality of longitudinally extending ceramic plates are configured to overlap, longitudinal side on longitudinal side upon retraction of the end effector into a sheath.
Clause 48. The end effector of any one of clauses 1-47, the end effector being configured to provide pulse field ablation electrical pulses having a voltage of about 600 volts and about 1,200 volts between pairs of ablation electrodes of the plurality of ablation electrodes.
Clause 49. The end effector of any one of clauses 1-48, the end effector being configured to provide a lesion depth of approximately 7.5 mm in a potato model.
Clause 50. The end effector of any one of clauses 1-49, the second ablation electrode being disposed on the second side of the planar body and non-overlapping with the first ablation electrode.
Clause 51. The end effector of any one of clauses 1-49, the second ablation electrode being disposed on the first side of the planar body and non-overlapping with the first ablation electrode.
Clause 52. The end effector of clause 50 or 51, the planar body having a left half and a right half as viewed on the first side, the left half being left of a centerline bisecting the planar body along the longitudinal axis, the right half being right of the centerline, the first ablation electrode defining a first electrode region disposed entirely in the left half, and the second ablation electrode defining a second electrode region disposed entirely in the right half.
Clause 53. The end effector of any one of clauses 1-49, the second ablation electrode being disposed on the second side of the planar body overlapping a majority of the first ablation electrode.
Clause 54. The end effector of any one of clauses 1-53, the plurality of ablation electrodes comprising exactly two, three, or four ablation electrodes on the first side of the planar body and exactly two, three, or four ablation electrodes on the second side of the planar body.
Clause 55. The end effector of clause 54, the plurality of ablation electrodes comprising exactly two ablation electrodes on the first side of the planar body and exactly two ablation electrodes on the second side of the planar body.
Clause 56. The end effector of clause 54, the plurality of ablation electrodes comprising exactly four ablation electrodes on the first side of the planar body and exactly four ablation electrodes on the second side of the planar body.
Clause 57. The end effector of any one of clauses 54-56, wherein the plurality of ablation electrodes that are on the first side of the planar body exactly overlap the plurality of ablation electrodes that are on the second side of the planar body.
Clause 58. A method of providing pulse field ablation electrical signals to a planar end effector, the method comprising: providing a first bipolar pulse field ablation electrical signal between a first ablation electrode and a second ablation electrode, the first ablation electrode being disposed on a first side of a planar body of the planar end effector, and the second ablation electrode being disposed on a second side of the planar body and non-overlapping with the first ablation electrode; and creating, with the planar end effector, a lesion with a depth of approximately 7.5 mm in a potato model.
Clause 59. The method of clause 58, wherein providing the first bipolar pulse field ablation electrical signal comprises providing pulses with a magnitude of approximately 1,200 volts for a duration of approximately 4 seconds.
Clause 60. The method of clause 58 or 59, wherein providing the first bipolar pulse field ablation electrical signal comprises providing 60 bursts of electrical pulses.
Clause 61. The method of any one of clauses 58-60, further comprising: providing a second bipolar pulse field ablation electrical signal between a third ablation electrode and a fourth ablation electrode, the third ablation electrode being disposed on a first side of a planar body of the planar end effector and overlapping with the second ablation electrode, and the fourth ablation electrode being disposed on a second side of the planar body and overlapping with the second ablation electrode.
Clause 62. The method of any one of clauses 58-61, further comprising: measuring cardiac electrical signals from a diagnostic electrode disposed on the first side of the planar body.
Clause 63. The method of any one of clauses 58-62, further comprising: measuring tissue contact from a pair of tissue contact electrodes disposed on the first side of the planar body.
Clause 64. The method of any one of clauses 58-63, wherein the bipolar pulses includes an interpulse delay of approximately 2 microseconds.

Having shown and described exemplary embodiments of the subject matter contained herein, further adaptations of the methods and systems described herein may be accomplished by appropriate modifications without departing from the scope of the claims. For instance, the end effector 100 may have an alternative shape or include alternative materials and the electrical signals used to achieve PFA may be modified to adapt to the specific geometry and materials of the end effector 100. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. An end effector of a medical probe, the end effector comprising:
a planar body comprising a first side and a second side opposite the first side and extending along a longitudinal axis; and
a plurality of ablation electrodes and comprising a first ablation electrode disposed on the first side of the planar body and a second ablation electrode,
the end effector being configured to provide bipolar pulse filed ablation electrical signals between the first ablation electrode and the second ablation electrode,
the first ablation electrode and the second ablation electrode each having a respective length of at least half of a total length of the end effector as measured from a distal end of the end effector to a distal end of a shaft of the medical probe such that the respective length of the first ablation electrode and the second ablation electrode define a distal portion of the end effector.

2. The end effector of claim 1, further comprising:
at least one diagnostic electrode disposed on at least one of the first side or second side of the planar body, the at least one diagnostic electrode being electrically isolated from the plurality of ablation electrodes and configured to receive electrical signals from tissue, optionally the at least one diagnostic electrode comprises plural pairs of diagnostic electrodes with each pair being disposed diametrically along an axis orthogonal to the longitudinal axis with respect to each other on respective first and second sides.

3. The end effector of any preceding claim, further comprising:
a plurality pairs of tissue contact electrodes spaced apart along the longitudinal axis, each pair of the plurality of pairs of tissue contact electrodes being disposed on at least one of the first side or the second side proximate the distal portion of the end effector.

4. The end effector of claim 1, comprising:
a plurality of first ablation electrodes comprising four individual first ablation electrodes, said first ablation electrode being one of the four individual first ablation electrodes, in which each of the four first ablation electrodes can be connected to the other three ablation electrodes to form one or more combined first ablation electrodes with a greater electrode area than any individual first ablation electrode; and
a plurality of second ablation electrodes comprising four individual second ablation electrodes, said second ablation electrode being one of the four individual second ablation electrodes, in which each of the four second ablation electrode can be connected to any of the other three second ablation electrodes to form a combined second ablation electrode with a greater electrode area than any individual second ablation electrode.

5. The end effector of claim 1, the first ablation electrode comprising a plurality of longitudinally elongated segments positioned left or right of each other, each segment of the plurality of longitudinally extending segments defining a width and a length, wherein a diagnostic electrode is positioned entirely within the width and entirely within the length of a respective longitudinally extending segment of the first ablation electrode.

6. The end effector of any preceding claim, wherein each ablation electrode of the plurality of ablation electrodes comprises a serpentine shape, wherein the serpentine shape of the first ablation electrode bends around at least three of four sides of a diagnostic electrode.

7. The end effector of any preceding claim, the plurality of ablation electrodes each being flush with the planar body to provide a first planar surface to the end effector corresponding to the first side of the planar body and a second planar surface to the end effector corresponding to the second side of the planar body.

8. The end effector of claim 1,
the planar body being symmetric about a centerline bisecting the planar body along the longitudinal axis,
the planar body having a left half and a right half as viewed on the first side,
the left half being left of the centerline,
the right half being right of the centerline, and
the first ablation electrode defining a first electrode region disposed entirely in the left half.

9. The end effector of claim 8, further comprising:
a pair of tissue contact electrodes disposed on the first side of the planar body across the centerline; and
a reference electrode disposed on the first side of the planar body across the centerline.

10. The end effector of claim 8, the plurality of ablation electrodes further comprising a third ablation electrode and a fourth ablation electrode, the end effector being configured to provide bipolar pulse filed ablation electrical signals between the third ablation electrode and the fourth ablation electrode.

11. The end effector of claim 10, the first, second, third, and fourth ablation electrodes having approximately equal surface area to each other.

12. The end effector of claim 10 or claim 11, each of the first, second, third, and fourth ablation electrodes defining a respective electrode region, each of the respective electrode regions begin disposed entirely in the right half of the planar body or entirely in the left half of the planar body.

13. The end effector of any preceding claim, the planar body comprising a planar high dielectric layer overlapping and parallel to each of the plurality of ablation electrodes.

14. The end effector of claim 13, the planar high dielectric layer comprising ceramic doped polymer and/or the planar body comprising longitudinally elongated regions comprising the planar high dielectric layer, lacking a framework, and lacking electrical circuitry.

15. The end effector of claim 1, the planar body comprising a plurality of longitudinally extending ceramic plates being angled such that the plurality of longitudinally extending ceramic plates are configured to overlap, longitudinal side on longitudinal side, upon retraction of the end effector into a sheath.

16. The end effector of any preceding claim, the end effector being configured to provide pulse field ablation electrical pulses having a voltage of about 600 volts and about 1,200 volts between pairs of ablation electrodes of the plurality of ablation electrodes.

17. The end effector of claim 1,
the second ablation electrode being disposed on the second side of the planar body and non-overlapping with the first ablation electrode,
the planar body having a left half and a right half as viewed on the first side,
the left half being left of a centerline bisecting the planar body along the longitudinal axis,
the right half being right of the centerline,
the first ablation electrode defining a first electrode region disposed entirely in the left half, and
the second ablation electrode defining a second electrode region disposed entirely in the right half.

18. The end effector of any preceding claim, the plurality of ablation electrodes comprising exactly two, three, or four ablation electrodes on the first side of the planar body and exactly two, three, or four ablation electrodes on the second side of the planar body.
